# EUROPEAN PATENT SPECIFICATION

(11) **EP 0 284 076 B1**
(45) Date of publication and mention of the grant of the patent: **08.06.1994**
(21) Application number: 88104768.2
(22) Date of filing: 24.03.1988
(51) Int. Cl.: C07D 207/32, C07D 487/04

(54) **Process for preparing (+)-1,2-dihydro-3H-pyrrolo[1,2-a]pyrrole-1-carboxylic acid and related compounds**
Verfahren zur Herstellung von (+)-1,2-Dihydro-3H-pyrrolo[1,2-a]-pyrrol-1-carbonsäure und verwandten Verbindungen
Procédé pour la préparation de l'acide (+)-1,2-dihydro-3H-pyrrolo-[1,2-a]pyrrole-1-carboxylique et des composés similaires

(30) Priority: 25.03.1987 US 30774
(43) Date of publication of application: 28.09.1988
(73) Proprietor: SYNTEX (U.S.A.) INC., Palo Alto California 94303 (US)
(72) Inventor: Fleming, Michael P., Longmont, Colorado 80501 (US); Schloemer, George C., Longmont, Colorado 80501 (US); Khatri, Hiralal N., Louisville, Colorado 80027 (US)
(74) Representative: Barz, Peter, Dr.

(56) References cited:
- EP-A- 0 041 711
- EP-A- 0 053 021
- DE-A- 2 731 662
- DE-A- 2 731 678
- US-A- 4 097 579
- US-A- 4 536 512
- CHEMICAL ABSTRACTS, vol. 101, no. 19, 5th November 1984, page 681, abstract no. 171016t, Columbus, Ohio, US; J.M. MUCHOWSKI et al.: "Lithiation of pyrrole-2-acetic acids. Synthesis of 2-alkylpyrroles"
- CHEMICAL ABSTRACTS, vol. 98, no. 1, 3rd January 1983, page 401, abstract no. 4471a, Columbus, Ohio, US; H. CARPIO et al.: "Sythesis of 1,2-dihydro-3H-pyrrolo(1,2-a)pyrrole-1-carboxylic acids and homologous pyridine and azepine analogs thereof"
- CHEMICAL ABSTRACTS, vol. 98, no. 25, 20th June 1983, page 545, abstract no. 215437g, Columbus, Ohio, US; E. GALEAZZI et al.: "Sythesis of ethyl 1,2-dihydro-3H-pyrrolo(1,2-a)pyrrole-1-carboxylate by an intramolecular carbenoid reaction",

## Description

This invention relates to pyrrolo[1,2-a]pyrroles, and especially to the synthesis of (±)-1,2-dihydro-3H-pyrrolo[1,2-a]pyrrole-1-carboxylic acid and related compounds.

5-Aroyl-1,2-dihydro-3H-pyrrolo[1,2-a]pyrrole-1-carboxylic acids, also known as 5-aroyl-1,2-dihydro-3H-pyrrolizine-1-carboxylic acids, of formula I, and the 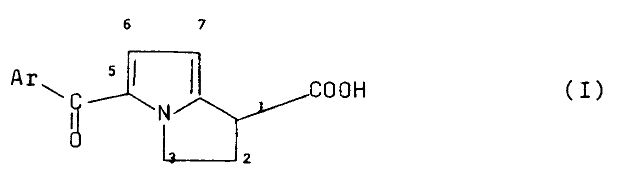
pharmacologically acceptable salts and esters thereof, are useful as analgesic, anti-inflammatory, and anti-pyretic agents for mammals, including man. They are also smooth muscle relaxants. Two exemplary compounds under clinical study in man are ketorolac, 5-benzoyl-1,2-dihydro-3H-pyrrolo[1,2-a]pyrrole-1-carboxylic acid, (I, Ar = C₆H₅), and anirolac, 5-p-anisoyl-1,2-dihydro-3H-pyrrolo[1,2-a]pyrrole-1-carboxylic acid, (I, Ar = p-CH₃O-C₆H₅), both disclosed in U S-A-4 089 969 to Muchowski et al. Other compounds, where the 5-aroyl substituents are substituted or unsubstituted benzoyl, furoyl, thenoyl, and pyrroyl, and where the 6-position on the pyrrolo-pyrrole nucleus is optionally substituted by lower alkyl or halogen, and the uses thereof, are also disclosed in a series of patents assigned to Syntex (U.S.A.) Inc., beginning with U S -A- 4 089 969, and including U S -A-4 087 539; 4 097 579; 4 140 698; 4 232 038; 4 344 943; 4 347 186; 4 458 081; 4 347 187; 4 454 326; 4 347 185; 4 505 927; 4 456 759; 4 353 829; 4 397 862; 4 457 941; and 4 454 151. U S -A- 4 511 724 and 4 536 512, assigned to Merck & Co., Inc., disclose 5-(substituted pyrrol-2-oyl)-1,2-dihydro-3H-pyrrolo[1,2-a]pyrrole-1-carboxylic acid derivatives and 5-(1,2-dihydro-3H-pyrrolo[1,2-a]pyrrol-2-oyl)-1,2-dihydro-3H-pyrrolo[1,2-a]pyrrole-1-carboxylic acid derivatives, respectively; while U S 4 533 671, also assigned to Merck & Co., Inc., discloses 5-(1,2-dihydro-3H-pyrrolo[1,2-a]pyrrol-2-oyl)-2-pyrrole-alkanoic acids and analogs.

Various methods for the preparation of these pyrrolo-pyrroles are exemplified in the patent and chemical literature, and many proceed through a common intermediate, 1,2-dihydro-3H-pyrrolo[1,2-a]pyrrole-1-carboxylic acid, (II), or its alkyl ester; 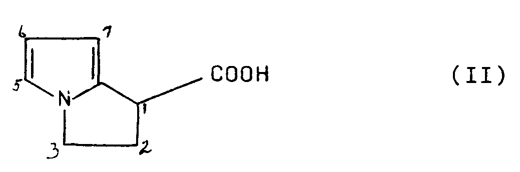
The alkyl ester may be readily 5-aroylated by methods such as those described in the previously-cited patents and in U S -A- 4 496 741 to Doherty, and saponified, to yield a 5-aroyl-1,2-dihydro-3H-pyrrolo[1,2-a]pyrrole-1-carboxylic acid (I).

Several methods of preparation of compound (II) are known in the patent and chemical literature, with most proceeding through 1,2-dihydro-3H-pyrrolo[1,2-a]pyrrole-1,7-dicarboxylic acid, which is selectively decarboxylated, by methods also described in the literature, to the 1-carboxylic acid.

Syntheses not involving the 1,7-dicarboxylate include those set forth in, for example, U S -A-4 140 698 and 4 344 943, referred to previously. These syntheses involve the preparation of 1-(2-iodoethyl)-pyrrole-2-acetonitrile (III, Z = CN) or an alkyl 1-(2-iodoethyl)-pyrrole-2-acetate (III, Z = COOR) and coupling to form the saturated ring, giving compound (IV). 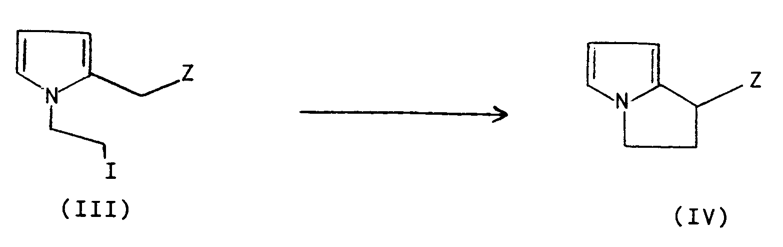
U S -A- 4 347 186 discloses a synthesis of (I) by the cyclization of a 1-[3,3-di(alkoxycarbonyl)propyl]-2-methanesulfonyl-5-aroylpyrrole (V) to the 1,1-dicarboxylate (VI), followed by hydrolysis to the 1-carboxylate. 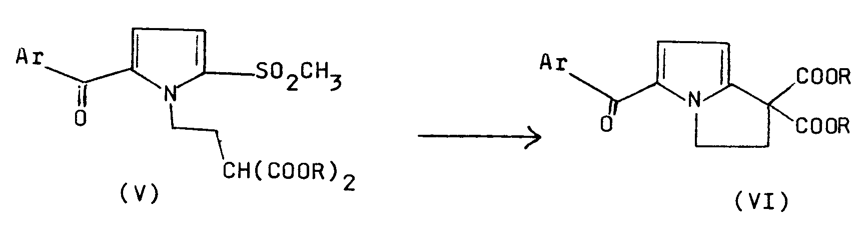
In each of these patents, the saturated ring is formed wholly or partially by the N-substituent on the pyrrole.

Pizzorno et al., in J. Org. Chem., 39, 731 (1974), disclose the cycloaddition of ethyl propiolate to N-formyl-L-proline (VII) to yield ethyl 1,2-dihydro-3H-pyrrolo[1,2-a]pyrrole-7-carboxylate (VIII), which is subsequently reduced to the corresponding pyrrolizidine carboxylate. 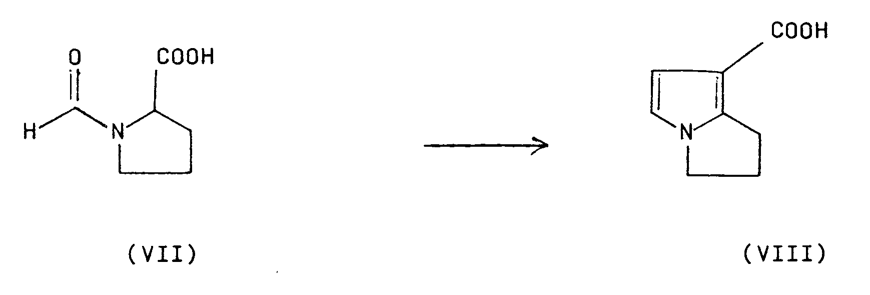
GB-A-1 234 139 discloses compounds including 7-hydroxymethyl- and 7-formyl-1,2-dihydro-3H-pyrrolo[1,2-a]pyrrole, prepared from the alkaloid derivative supinidine.

In a first aspect, this invention relates to the preparation of (±)-1,2-dihydro-3H-pyrrolo[1,2-a]pyrrole-1-carboxylic acid compounds of formula (IX), 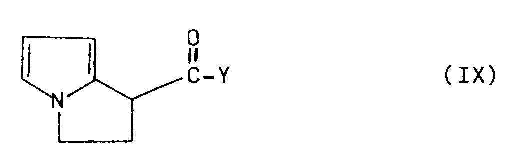
in which
- Y is: OH;
O⁻M⁺, wherein M is an alkali metal; or
NRR', wherein R is lower alkyl and R' is lower alkyl or aryl, or NRR' is the residue of a saturated cyclic amine,
from pyrrole.

The preparation may be represented schematically: 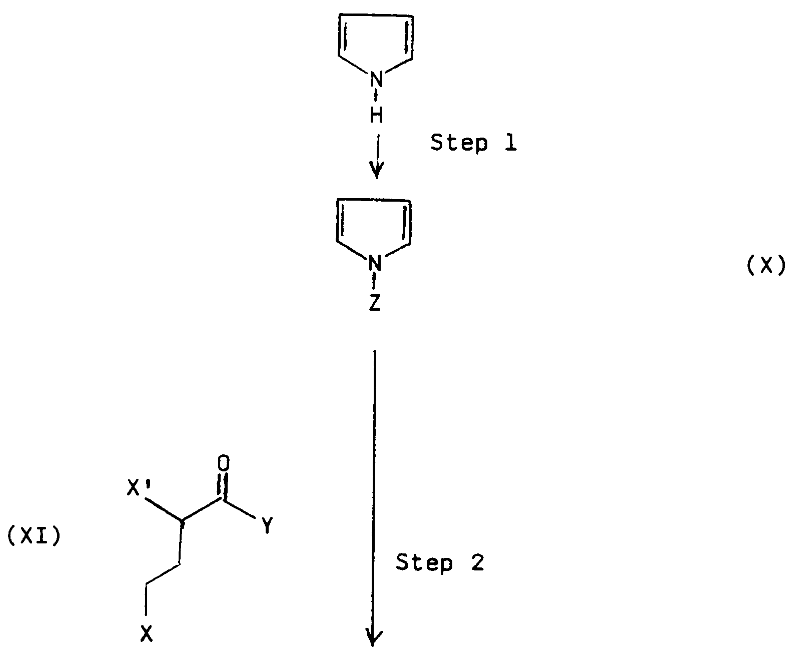 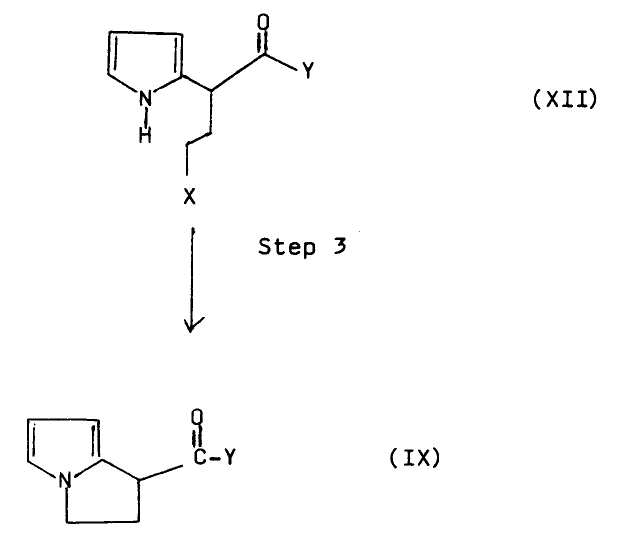
in which
- Y is: as previously defined;
- X and X': are independently halogen; and
- Z is: Li, MgCl, or MgBr.

In a second aspect, this invention relates to a process for preparing (±)-1,2-dihydro-3H-pyrrolo[1,2-a]pyrrole-1-carboxylic acid (IX, Y = OH), which comprises preparing a compound of formula (IX), where Y is other than OH, by the process described above, followed by hydrolysis thereof, or preparing an ester thereof (IX, Y = OR) by preparing the acid (IX, Y = OH), followed by esterification.

In a third aspect, this invention relates to novel compounds of formula (XII) which are useful as intermediates in the process herein.

In a fourth aspect this invention relates to the process for preparing compound of formula (I) or (IA) from compounds of formula (XII), which comprises the further steps of
(a) reacting a compound of formula (XII) 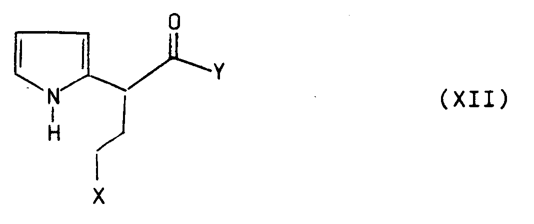 in which
   - Y is: OH,
   O⁻M+, wherein M is an alkali metal, or
   NRR', wherein R is lower alkyl and R' is lower alkyl or aryl, or NRR'' is the residue of a saturated cyclic amine; and
   - X is: halogen,
   with a strong base in the aprotic polar solvent to form a compound of formula (IX) 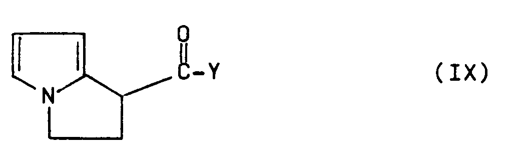 in which
   - Y is: OH;
   O⁻M⁺, wherein M is an alkali metal; or
   NRR', wherein R is lower alkyl and R' is lower alkyl or aryl, or NRR' is the residue of a saturated cyclic amine;
(b) hydrolyzing, if applicable, a compound of formula (IX) wherein Y is O⁻M⁺ or NRR' to afford a compound of formula (IX) wherein Y is OH;
(c) esterifying a compound of formula (IX) wherein Y is OH to form a compound of formula (IX) wherein Y is OR wherein R is alkyl of 1 to 12 carbon atoms;
(d) aroylation of an ester of formula (IX) with an amide or morpholide, if appropriate, followed by hydrolysis to form a compound of formula (I) or (IA) 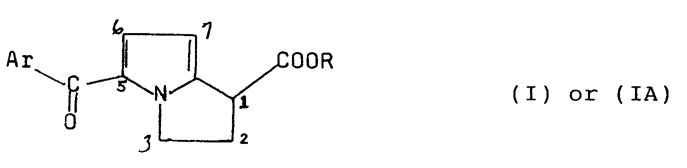 wherein Ar is substituted or unsubstituted phenyl, 2- or 3-furyl, 2- or 3-thienyl, or 2- or 3-pyrryl which, if substituted, can have one or more lower alkyl, lower alkoxy, or halo groups in any available position on the ring, and R is hydrogen (formula I) or alkyl of 1 to 12 carbon atoms (formula IA).

A further aspect this invention relates to the further process of one or more of the steps:
(a) converting a compound of formula (I) to a pharmaceutically acceptable salt of a compound of formula (I); or
(b) converting a salt of a compound of formula (I) to the corresponding free compound of formula (I); or
(c) esterifying a compound of formula (I); or
(d) converting an ester of formula (IA) to the corresponding free compound of formula (I).

A further aspect of this invention relates to the process for the preparation of 5-benzoyl-1,2-dihydro-3H-pyrrolo[1,2-a]pyrrole- 1-carboxylic acid or a pharmaceutically acceptable salt or ester thereof.

A further aspect of this invention relates to the process for the preparation of 5-p-anisoyl-1,2-dihydro-3H-pyrrolo[1,2-a]- pyrrole-1-carboxylic acid or a pharmaceutically acceptable salt or ester thereof.

### Definitions

Unless otherwise stated, the following terms used in the specification and claims have the meanings given below:
"lower alkyl", denoted generally by R or R', refers to straight, branched, or cyclic saturated hydrocarbon radicals having from one to six carbon atoms, e.g. methyl, ethyl, n-propyl, isopropyl, n-butyl, t-butyl, cyclopropylmethyl, pentyl, cyclohexyl, hexyl. Preferred lower alkyls are methyl, ethyl, and n-propyl, and a particularly preferred lower alkyl is methyl. If more than one alkyl radical is present in a given molecule, each may be independently selected from "lower alkyl" unless otherwise stated.
"lower alkoxide", "lower alkanol", "lower alkylamine", "lower alkyl ester", and similar terms refer to alkoxides, alkanols, alkylamines, alkyl esters, in which the (or each) alkyl radical is a "lower alkyl" as defined above.
"aryl", denoted generally by Ar, refers to a substituted or unsubstituted monovalent unsaturated radical, for example, phenyl, 2- or 3- furyl, 2- or 3-thienyl, or 2- or 3- pyrryl, which, if substituted, can have one or more lower alkyl, lower alkoxy, or halogen groups (e.g. 4-tolyl, 4-fluorophenyl) in any available position on the ring. Phenyl and p-methoxyphenyl are preferred.
"halogen", denoted generally by X or X', refers to chlorine, bromine, or iodine. Preferred halogens are chlorine and bromine.
"aprotic polar solvent" includes organic solvents which may be either water-immiscible, such as halogenated hydrocarbons, e.g. methylene chloride, chloroform, or water-miscible, such as tetrahydrofuran, dimethoxyethane, bis(2-methoxyethyl) ether (also known as diglyme), dimethylformamide, N-methylpyrrolidone, dimethylsulfoxide. The solvent may also contain minor proportions of aprotic non-polar solvents such as hydrocarbons, e.g. cyclohexane, toluene, provided that the solvent properties are largely determined by the polar solvent.
"residue of a saturated cyclic amine" refers to the residue (i.e. that part other than the N-bonded hydrogen atom) of a saturated cyclic amine containing 5 or 6 ring atoms, e.g. pyrrolidine, piperidine, piperazine, morpholine.
"alkali metal" refers to electropositive metals including lithium, sodium, potassium, rubidium, and cesium.
"strong base" refers to bases such as alkali metal hydroxides, lower alkoxides, hydrides, di(lower alkyl)amines, e.g. sodium hydroxide, potassium methoxide, sodium hydride, lithium di(isopropyl)amine, lithium bis(trimethylsilyl)amine.
"weak base" refers to the alkali metal or alkaline earth salt of a weak acid, e.g. sodium acetate, potassium bicarbonate, or to a buffer mixture (such as NaH₂PO₄/Na₂HPO₄) giving a similar pH.
"strong base" refers to bases such as alkali metal hydroxides, lower alkoxides, hindered amines such as di(lower alkyl)amines and bis(tri lower alkylsilyl) amines, hydrides, which contain alkali metals including lithium, sodium, potassium, rubidium, and cesium, e.g. sodium hydroxide, potassium hydroxide, potassium methoxide, sodium methoxide, potassium ethoxide, sodium ethoxide, sodium hydride, lithium di(isopropyl)amine, lithium bis(trimethylsilyl)amine.
"pharmaceutically acceptable salt" refers to salts derived from inorganic bases which include sodium, potassium, lithium, ammonium, calcium, magnesium, ferrous, zinc, copper, manganous, aluminum, ferric and manganic salts. Particluarly preferred are the ammonium, potassium, sodium, calcium, and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-di-ethylaminoethanol, tromethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine and polyamine resins. Particularly preferred organic non-toxic bases are isopropylamine, diethylamine, ethanolamine, piperidine, tromethamine, dicyclohexylamine, choline, and caffeine.
"pharmaceutically acceptable esters" refers to alkyl esters derived from hydrocarbons of branched or straight chain having from one to twelve carbon atoms. Typical alkyl ester groups are, for example, methyl, ethyl, propyl, isopropyl, butyl, t-butyl, isoamyl, pentyl, isopentyl, hexyl, octyl, nonyl, isodecyl, 6-methyldecyl, and dodecyl.

### Starting Materials and Purification

The starting pyrrole and compounds of formula (XI), the products of formula (IX), and the intermediates of formulae (X) and (XII), may be isolated and purified, if desired, using conventional techniques, including but not limited to filtration, distillation, crystallization and chromatography. They may be characterized using conventional means, including physical constants and spectral characteristics.

### Preparation of Compounds of Formula XI

The 2-X'-4-X-butanoic acid derivatives of formula (XI) may be prepared by methods known to the art. See, for example, DE-C- 804 567 (BASF), for the preparation of 2-bromo-4-chloro- and 2,4-dichlorobutanoyl chloride. Other acid halides may be prepared in a similar manner, and the acid halides may be converted to the desired amides or acid salts by methods known to the art, e.g., reaction with a diamine or hydrolysis.

### Preparation of Compounds of Formula IX

In Step 1, the pyrrole is lithiated or converted to a Grignard reagent by methods well known to the art. For example, 1-lithiopyrrole is readily prepared by the reaction of pyrrole, typically in solution in an aprotic solvent such as a di(lower alkyl) ether, tetrahydrofuran, and the like, with an alkyllithium, e.g. n-butyllithium in hexane or similar solution, at a temperature between about 0 and 50°C, generally at reduced temperatures, e.g., near 0°C. 1-pyrrolemagnesium chloride (or bromide) is readily prepared by the reaction of pyrrole, again typically in solution in a aprotic solvent, with an alkylmagnesium chloride (or bromide), typically also in solution in an aprotic solvent, e.g. methylmagnesium chloride in tetrahydrofuran (THF), generally at reduced temperatures. The reactions are generally performed under an inert atmosphere, e.g. nitrogen, and occur rapidly, typically within 10 minutes. The resulting 1-lithiopyrrole or 1-pyrrolemagnesium chloride/bromide solutions are generally used immediately in Step 2.

In Step 2, the 2-X'-4-X-butanoic acid derivative of formula (XI) is treated with the pyrrole reagent (X) from Step 1 until reaction is complete. The pyrrole reagent (X) is preferably present in excess, for example between 1.2-fold and 3-fold excess, typically about a 2-fold excess, over compound (XI) to minimize the formation of 2,5-disubstituted pyrrole products. The reaction is generally performed under an inert atmosphere, by addition of a solution of compound (XI) in an aprotic polar solvent to a stirred solution of the pyrrole reagent (X), e.g. the solution from Step 1. The reaction temperature may be from about 0 to 60°C, but the reaction is slowed by cooling, and addition typically takes place at reduced temperatures, e.g. near 0°C, in one or more portions; after which the reaction mixture is allowed to warm to up to about 40°C, preferably warm up to room temperature (about 15 to 25°C), allowing the reaction to proceed to completion. The reaction time may range from 30 minutes to 48 hours, but is ordinarily about 5 to 20 hours. Following completion of the reaction (the progress of which may be checked by methods such as thin layer chromatography, etc.), the resulting intermediates of formula (XII) may be isolated by conventional techniques, such as addition of water and acidification of the solution with mineral acid, e.g. by addition of concentrated hydrochloric acid to pH 1, followed by extraction into a suitable organic solvent, e.g ether, and evaporation of the solvent. They may be purified most readily by chromatography.

Cyclization to the pyrrolo[1,2-a]pyrrole derivative of formula (IX) takes place in Step 3. Here, the intermediate of formula (XII) is dissolved in an aprotic polar solvent, e.g. THF, and treated with an excess of a strong base such as an alkali metal hydride, preferably sodium hydride. The reaction takes place typically at room temperature (about 15 to 25°C), in about 10 minutes to 10 hours, preferably in about 1 to 5 hours, under an inert atmosphere. The excess hydride is destroyed, e.g. by slow addition of water, and the product of formula (IX) may then be recovered, e.g. for the amide by addition of a suitable organic solvent, such as ether, washing, and removal of the solvent; for the acid, by acidification, extraction of the acid into an organic phase, and evaporation of the solvent.

### Conversion of the Acid/Ester of Formula IX

The amide of formula (IX), where Y is NRR', may be converted into the acid by either acid- or base-catalyzed hydrolysis. For example, the amide may be treated with a dilute solution of a strong mineral acid, such as sulfuric acid, generally at elevated temperatures, e.g. up to reflux temperature, until hydrolysis is complete, and the resulting acid (IX, Y = OH) recovered in the manner described above. Alternatively, the amide may be treated with a solution of a strong base, such as an alkali metal hydroxide, typically in a protic organic solvent, generally at elevated temperatures, until hydrolysis is complete; water added and the resulting solution acidified; and the resulting acid (IX, Y = OH) separated, e.g. by extraction into an immiscible organic solvent. The acid of formula (IX), obtained either in Step 3 or by the conversion just described, may be converted to a desired ester by conventional esterification techniques, e.g. reaction with an alcohol in the presence of an acid catalyst.

### Preparation of Compounds of Formula I

Esters of compound (II, Y = OR) may be converted into the corresponding 5-aroyl-1,2-dihydro-3H-pyrrolo[1,2-a]pyrrole-1-carboxylic acids by 5-aroylation, followed by hydrolysis. The 5-aroylation may be performed by methods known to the art, e.g. Vilsmeier-Haack or Friedel-Crafts aroylations, and described in the patents cited in he "Background to the Invention" section of this application, especially U.S. Patents Nos. 4,089,969 and 4,347,186 (using dialkylamides) and U.S. Patent No. 4,353,829 (using morpholides), and the hydrolysis may be performed as described above.

Condensation of an ester of formula (II) can be accomplished in one of two ways, with an amide or with a morpholide.

Condensation of an ester of formula (II) with an amide of the formula 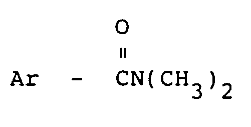
wherein Ar is one of the substituents defined above, affords the corresponding alkyl 5-aroyl-1,2-dihydro-3H-pyrrolo[1,2-a]pyrrole-1-carboxylate (IA). This reaction is conducted in an inert organic aprotic solvent and in the presence of phosphorous oxychloride, at reflux temperature for from about 1 to about 175 hours, under an inert atmosphere, followed by further reflux in the presence of sodium acetate, for from about 2 to about 10 hours. Alternatively, instead of phosphorous oxychloride, other acid chlorides such as phosgene or oxalyl chloride may be used. In the preferred embodiment, this condensation is carried out by adding a solution of ester of (II) in a suitable solvent to a previously refluxed mixture of 1.1 to 5 molar equivalents of both the desired amide and phosphorous oxychloride in the same solvent, refluxing the reaction mixture thus obtained for from about 6 to about 72 hours under an argon atmosphere and thereafter adding thereto from about 3 to about 10 molar equivalents of sodium acetate, followed by an additional reflux period for from about 4 to about 6 hours. Adequate solvents for this reaction are the halogenated hydrocarbons such as dichloromethane, 1,2-dichloroethane, chloroform and carbon tetrachloride, dimethoxyethane and tetrahydrofuran. The preferred solvent is 1,2-dichloroethane.

Representative of the N,N-dimethyl arylamides which can be used are: N,N-dimethyl-benzamide, N,N-dimethyl-o-toluamide, N,N-dimethyl-m-toluamide, N,N-dimethyl-p-toluamide, N,N-dimethyl-p-ethyl-benzamide, N,N-dimethyl-o-propyl-benzamide, N,N-dimethyl-m-butyl-benzamide, N,N-dimethyl-o-methoxy-benzamide, N,N-dimethyl-m-methoxy-benzamide, N,N-dimethyl-p-methoxy-benzamide, N,N-dimethyl-p-ethoxy-benzamide, N,N-dimethyl-p- isopropoxy-benzamide, N,N-dimethyl-o-chloro-benzamide, N,N-dimethyl-m-chloro-benzamide, N,N-dimethyl-p-chloro-benzamide, N,N-dimethyl-o-fluoro-benzamide, N,N-dimethyl-p-fluro-benzamide, N,N-dimethyl-m-bromo-benzamide, and N,N-dimethyl-p-bromo-benzamide. These amides are known, commercially available compounds or can be prepared in a conventional manner from the corresponding acids, i.e., by conversion into the acid chlorides followed by treatment with dimethylamine.

If an aroyl morpholide is used, the ester of formula (II) is treated with an aroyl morpholide of the formula 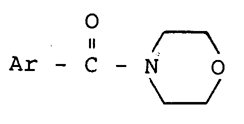
wherein Ar is as defined above, in the presence of an inorganic acid halide, such as POCl₃, POBr₃, SO₂Cl₂ and the like, preferably POCl₃. The relative amounts are not critical. Optionally an inert organic solvent such as ethane dichloride, chloroform, or carbon tetrachloride, but preferably methylene chloride, may be included in this mixture. The presence of the solvent, however, is not particularly advantageous in all cases. The mixture is agitated, preferably stirred, for about 0.5 to 36 hours, preferably 1 to 5 hours at about 30 to 50°C, preferably 40 to 45°C.

A solution of the pyrrolo pyrrole substrate of formula (II) in an inert solvent, most preferably CH₂Cl₂, is then added to the above mixture. Again the ratio of reactants is not critical, but it is preferable that the molar amount of the substrate be slightly less than the molar amount of the minority reactant in the prepared morpholide/halide mixture. The resulting reaction mixture is kept at about 30 to 70°C, preferably 40 to 45°C until the desired reaction has taken place, usually about 1 to 8 hours, must usually 1.5 to 3 hours.

The entire procedure to this point is carried out in an inert atmosphere in order to exclude water. Any anhydrous gas could be used, but nitrogen is the most convenient choice. As the reaction is scaled up, the problem of water in the ambient air becomes smaller because of less proportional available surface area. However, it has been found prudent to use nitrogen as a matter of routine.

The intermediate formed at this point cannot conveniently be isolated, but must be hydrolyzed either to the ester of formula (IA), or to the free acid of formula (I).

If it is desired to prepare a compound of formula (I), a single step procedure is preferred. In this embodiment, the reaction mixture is poured into a solution in polar solvent, preferably aqueous, of a strong base, such as a mineral hydroxide or carbonate, preferably sodium hydroxide. A large excess of the base is used. The mixture is then kept at about 30 to 100°C, preferably 40 to 60°C until reaction is complete.

Alternatively, if the two-step procedure is to be used, or a compound of formula (IA) is to be prepared, an amount of from about 3 to about 10 molar equivalents of sodium acetate or other weak base may be added directly to the reaction mixture, followed by an additional reaction time of about 4 to 6 hours during which the mixture is refluxed. At the end of this period, the compound of formula (IA) is produced.

If subsequent conversion to the compound of formula (I) is desired, further hydrolysis is carried out in a conventional manner with an alkali metal hydroxide or carbonate in an aqueous or aqueous lower aliphatic alcohol (e.g. methanol or ethanol) solution. The temperature is about room temperature to reflux and the reaction time about 15 minutes to about 3 hours. Preferably, the hydrolysis is effected with aqueous methanolic potassium carbonate at reflux temperatures for about 30 minutes.

Upon alkaline hydrolysis of the alkyl ester group in a compound of formula (IA) there is obtained the corresponding free acid of formula (I). This hydrolysis is effected in a conventional manner, with an alkali metal hydroxide or alkali metal carbonate, e.g., sodium hydroxide, potassium hydroxide, sodium carbonate, potassium carbonate, in an aqueous lower aliphatic alcohol, e.g., methanol, ethanol, at a temperature of from about room temperature to reflux, for from about 15 minutes to about 2 hours, under an inert atmosphere. In the preferred embodiment, this hydrolysis is effected with aqueous methanolic potassium carbonate, at reflux temperature for about 30 minutes.

The free acids of formula (I) can be converted into other alkyl esters having from 1 to 12 carbon atoms by conventional methods, e.g., by treatment with (a) the alcohol corresponding to the desired ester in the presence of a strong mineral acid, (b) an etheral diazoalkane or (c) the desired alkyl iodide in the presence of lithium carbonate.

The salt derivatives of the compounds of formula (I) are prepared by treating the free acids with an appropriate amount of a pharmaceutically acceptable base. The reaction is conducted in water, alone or in combination with an inert, water-misible organic solvent, at a temperature of from about 0°C to about 100°C, preferably at room temperature. Typical inert, water-misible organic solvents include methanol, ethanol, isopropanol, butanol, acetone, dioxane, or tetrahydrofuran. The molar ratio of compounds of formula (I) to base used are chosen to provide the ratio desired for any particular salt.

It should be noted that all of these methods to afford compounds of formula I are set forth in, e.g. U S -A-4 089 969 and 4 353 829.

### Novel Intermediates

The compounds of formula (XII) are novel, and are useful as intermediates in preparing compounds of formula (I), which are therapeutically useful as discussed above.

### EXAMPLES

The invention is further illustrated by the following examples.

### Example 1: Preparation of 2-bromo-4-chlorobutanoyl chloride.

A. 2-Bromo-γ-butyrolactone was treated according to the procedure of West German Patent No. 804 567, to produce 2-bromo-4-chlorobutanoyl chloride. Thus, 2-bromo-γ-butyrolactone (Aldrich, 99.51 g, 603 mmol) and zinc(II) chloride (Alfa ultrapure, 8.45 g, 62 mmol) were added to a 250 mL three-neck flask containing a stirring bar and fitted with an addition funnel and a reflux condenser. The mixture was heated to 65°C with stirring, and thionyl chloride (82.43 g, 693 mmol) was added through the addition funnel over 1 hour. Since a sample taken after 16 hours showed substantial remaining lactone, the mixture was cooled briefly, additional zinc(II) chloride (5.43 g, 40 mmol) was added, and the mixture heated for an additional 44 hours at about 65°C. Dichloromethane (100 mL) was then added, the resulting solution rapidly filtered through a coarse glass frit, and the thionyl chloride and dichloromethane removed by evaporation under aspirator vacuum at about 30°C. Distillation of the residue through a short packed column gave 87.51 g (66% yield) of a yellow oil, having a boiling point under aspirator vacuum of 108 - 110°C. An NMR spectrum was consistent with 2-bromo-4-chlorobutanoyl chloride.
B. Substituting 2-chloro-γ-butyrolactone for 2-bromo-γ-butyrolactone, and using a similar procedure to that in part A of this Example, one obtains
   2,4-dichlorobutanoyl chloride.
C. Similarly, substituting thionyl bromide for thionyl chloride and zinc(II) bromide for zinc(II) chloride in the procedure of parts A and B of this Example, one obtains
   2,4-dibromobutanoyl bromide, and
   2-chloro-4-bromobutanoyl bromide.

### Example 2: Preparation of N,N-diethyl-2-bromo-4-chlorobutanamide.

A. 2-Bromo-4-chlorobutanoyl chloride (101.7 g, 463 mmol) was added to a 1 L three-neck flask fitted with a mechanical stirrer, addition funnel, and thermometer, and the flask then purged with nitrogen. Dichloromethane (400 mL) was added, and the flask cooled in an ice bath to 0°C. Diethylamine (Baker, 66.8 g, 913.5 mmol) was added dropwise over 75 minutes, with the temperature rising to 5 to 10°C, after which the ice bath was removed and the flask warmed to room temperature (about 20°C) with a warm water bath, and the reaction mixture stirred for 30 minutes at that temperature. Water (250 mL) was added to the mixture; and the organic phase was separated, washed with water (2 x 250 mL), aqueous KHCO₃ (2 x 100 mL), and brine (100 mL), and dried over anhydrous magnesium sulfate. Removal of the solvent at 50°C under reduced pressure gave a brown oil, which was distilled (Kugelrohr, 95°C, 0.15 mm) to afford 108.5 g (92% yield) of an extremely pale green oil, having an NMR spectrum consistent with N,N-diethyl-2-bromo-4-chlorobutanamide.
B. Substituting for 2-bromo-4-chlorobutanoyl chloride, in the procedure of part A of this Example,
   2,4-dichlorobutanoyl chloride,
   2,4-dibromobutanoyl bromide, or
   2-chloro-4-bromobutanoyl bromide,
   one obtains, respectively,
   N,N-diethyl-2,4-dichlorobutanamide.
   N,N-diethyl-2,4-dibromobutanamide.
   N,N-diethyl-2-chloro-4-bromobutanamide.
C. Similarly, substituting other di(lower alkyl)amine, aryl(lower alkyl)amines, or cyclic amines for diethylamine, one obtains N,N-di(lower alkyl)- or N-aryl-N-(lower alkyl)-2-halo-4-halobutanamides, 2-halo-4-halo-butanoylmorpholides, and the like.

### Example 3: Preparation of the pyrrole reagent. (Step 1)

A. Pyrrole (Eastman, distilled over CaH₂, 0.60 g, 8.95 mmol) and tetrahydrofuran (distilled over sodium, 20 mL) were added to a 100 mL flask containing a stirrer bar, under strictly anhydrous conditions, and the flask cooled in an ice bath. n-Butyllithium (Aldrich, 2.6 M in hexane, 3.0 mL, 7.80 mmol) was added via syringe over about 1 minute, and the clear solution was stirred for 5 minutes. The resulting solution of 1-lithiopyrrole was used in Step 2.
B. Pyrrole (Eastman, distilled over CaH₂, 1.49 g, 22.2 mmol) and tetrahydrofuran (distilled over sodium, 30 mL) were added to a 100 mL flask containing a stirrer bar, under strictly anhydrous conditions, and the flask cooled in an ice bath. Methylmagnesium chloride (3.0 M in THF, 7.2 mL, 21.6 mmol) was added via syringe over about 5 minutes, during which time the temperature rose to about 20°C, and the mixture was stirred for 15 minutes. The resulting white slurry containing 1-pyrrolemagnesium chloride was used in Step 2.
C. Substituting methylmagnesium bromide for methylmagnesium chloride in the procedure of part B of this Example, one obtains
   1-pyrrolemagnesium bromide.

### Example 4: Preparation of α-(2-chloroethyl)-N,N-diethyl-3H-pyrrole-2-acetamide. (Step 2)

A. To the solution of 1-lithiopyrrole from part A of Example 3, maintained at 0°C in the ice bath, was added N,N-diethyl-2-bromo-4-chlorobutanamide (1.09 g, 4.25 mmol) in a single portion via syringe. Within 30 minutes of stirring at 0°C, the initially yellow solution had become dark purple; and stirring at 0°C was continued for 2 hours. The solution was then allowed to warm to room temperature, and stirred for 17 hours. Water (10 mL) was then added, and the mixture acidified to pH 1 with concentrated HCl, then diluted with ether (150 mL), washed with water (2 x 20 mL), aqueous KHCO₃ (2 x 20 mL), and brine (20 mL), and stirred over MgSO₄ and Filtrol®. Rotary evaporation of the solvents under reduced pressure gave 0.72 g of a brown oil, which was shown by NMR spectra and thin-layer chromatography to contain a major proportion of α-(2-chloroethyl)-N,N-diethyl-3H-pyrrole-2-acetamide, (XII, X = Cl, Y = NEt₂), a minor proportion of N,N-diethyl-1,2-dihydro-3H-pyrrolo[1,2-a]pyrrole-1-carboxamide (IX, Y = NEt₂), and very minor proportions of the pyrrole-2,5-di(substituted acetamide) and unreacted N,N-diethyl-2-bromo-4-chlorobutanamide.
B. To the slurry containing 1-pyrrolemagnesium chloride from part B of Example 3, maintained at 0°C in the ice bath, was added N,N-diethyl-2-bromo-4-chlorobutanamide (2.77 g, 10.8 mmol) in 15 mL Na-distilled tetrahydrofuran over 2.75 hours via syringe pump. The reaction mixture was stirred for 30 minutes at 0°C, then allowed to warm to room temperature, and stirred for 19 hours, resulting in a brownish-black solution. Water (10 mL) was then added, and the mixture acidified to pH 1 with concentrated HCl, then diluted with ether (150 mL), washed with water (2 x 25 mL), aqueous KHCO₃ (2 x 25 mL), and brine (25 mL), and dried over MgSO₄. The black solution was stirred with DARCO® activated carbon for 15 minutes, and filtered through paper to give a light brown solution. Rotary evaporation of the solvents under reduced pressure gave 2.43 g of a viscous brown oil, which was shown by NMR spectra and thin-layer chromatography to contain a major proportion of α-(2-chloroethyl)-N,N-diethyl-3H-pyrrole-2-acetamide, (XII, X = Cl, Y = NEt₂), and minor proportions of the pyrrole-2,5-di(substituted acetamide) and unreacted N,N-diethyl-2-bromo-4-chloro-butanamide. Double Kugelrohr distillation (130 - 135°C, 0.15 mm) afforded α-(2-chloroethyl)-N,N-diethyl-3H-pyrrole-2-acetamide as a white solid having NMR (CDCl₃): δ: 8.8 - 9.2 (1H, broad singlet), 6.6 - 6.8 (1H, multiplet), 6.0 - 6.2 (2H, multiplet), 4.24 (1H, triplet), 3.2 - 3.6 (6H, multiplet), 2.1 - 2.4 (2H, multiplet), 1.0 - 1.3 (6H, multiplet).

### Example 5: Preparation of N,N,-diethyl-1,2-dihydro-3H-pyrrolo[1,2-a]pyrrole-1-carboxamide. (Step 3)

A. Sodium hydride (Aldrich, 50% in mineral oil, 0.140 g, 2.91 mmol) was added to a 25 mL flask containing a stirrer bar, and washed with dry tetrahydrofuran (distilled over sodium, 4 x 5 mL). 10 mL tetrahydrofuran was added, followed by α-(2-chloroethyl)-N,N-diethyl-3H-pyrrole-2-acetamide (0.115 g, 0.47 mmol). The resulting mixture was stirred at room temperature for 3 hours, by which point tlc showed no starting material. Four drops of water were slowly added, producing vigorous gas evolution, and the mixture diluted with ether (50 mL) while being transferred to a separator funnel. The solution was extracted with water (4 x 20 mL) and brine (20 mL), and dried over MgSO₄; and the solvents removed by vacuum rotary evaporation to afford 0.10 g of a light brown viscous oil, N,N-diethyl-1,2-dihydro-3H-pyrrolo[1,2-a]pyrrole-1-carboxamide (II, Y = NEt₂), having NMR (CDCl₃): δ: 6.58 - 6.61 (1H, multiplet), 6.17 (1H, triplet), 5.79 (1H, doublet), 2.8 - 4.3 (8H, multiplet), 2.4 - 2.8 (1H, multiplet), 1.26 (3H, triplet), 1.11 (3H, triplet).

Comparison of the NMR spectrum and R_{f} value with those of an authentic specimen of N,N-diethyl-1,2-dihydro-3H-pyrrolo[1,2-a]pyrrole-1-carboxamide prepared from the corresponding acid showed that the materials were identical.

### Example 6: Preparation of 1,2-dihydro-3H-pyrrolo[1,2-a]pyrrole-1-carboxylic acid.

N,N-Diethyl-1,2-dihydro-3H-pyrrolo[1,2-a]pyrrole-1-carboxamide is dissolved in ethylene glycol, and potassium hydroxide is added. The mixture is heated to 100°C for 8 hours, cooled, and water added. The resulting mixture is acidified with concentrated HCl and extracted with methylene chloride. The organic layer is evaporated, and the residue sublimed at 55-60°C/0.133 mbar (0.1 mmHg) to afford the title compound as a white solid.

### Example 7: Preparation of methyl 1,2-dihydro-3H-pyrrolo[1,2-a]-pyrrole-1-carboxylate.

1,2-Dihydro-3H-pyrrolo[1,2-a]pyrrole-1-carboxylic acid is dissolved in methanol containing a small amount of concentrated hydrochloric acid. The resulting solution is heated to reflux, and then cooled. The title compound is isolated by rotary evaporation of the solvent under reduced pressure, and may be purified by chromatography on silica gel with hexane/ethyl acetate as eluent.

### Example 8: Preparation of 5-aroyl-1,2-dihydro-3H-pyrrolo[1,2-a]pyrrole-1-carboxylic acids

A. 1,2-Dihydro-3H-pyrrolo[1,2-a]pyrrole-1-carboxylic acid is dissolved in isopropanol containing a small amount of concentrated hydrochloric acid. The resulting solution is heated to reflux, and then cooled. Isopropyl 1,2-Dihydro-3H-pyrrolo[1,2-a]pyrrole-1-carboxylic acid is isolated by rotary evaporation of the solvent under reduced pressure, and may be purified by chromatography on silica gel with hexane/ethyl acetate as eluent. In this manner there is obtained isopropyl 1,2-dihydro-3H-pyrrolo[1,2-a]pyrrole- 1-carboxylate, a pale yellow oil, having the following physical constants: 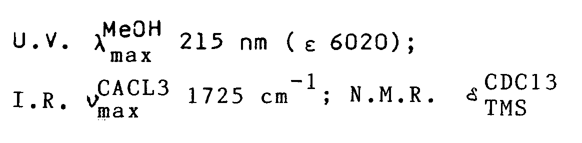 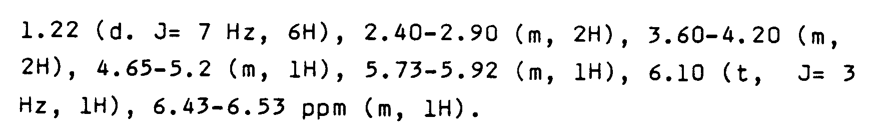
B. A solution of 179 mg of N,N-dimethyl-p-toluamide and 0.11 ml of phosphorous oxychloride in 2 ml of 1,2-dichloroethane is refluxed for 30 minutes. To this solution is added a solution of 193 mg of isopropyl 1,2-dihydro-3H-pyrrolo[1,2-a]pyrrole-1-carboxylate in 2 ml of 1,2-dichloroethane. The reaction mixture is refluxed under an argon atmosphere for 8 hours, treated with 405 mg of sodium acetate and refluxed for a further 5 hours. The resultant mixture is then evaporated to dryness and the residue is chromatographed on 12 g of silica gel, eluting with hexane:ethyl acetate (3:1), thus obtaining 208 mg (66%) of isopropyl 5-p-toluoyl-1,2-dihydro-3H-pyrrolo[1,2-a]pyrrole-1-carboxylate, an oil, having the following physical constants: 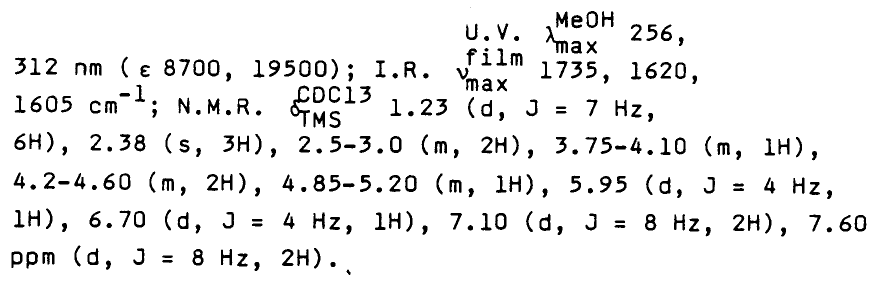
C. A solution of 336 mg of isopropyl 5-p-toluoyl-1,2-dihydro-3H-pyrrolo[1,2-a]pyrrole-1-carboxylate in 10 ml of methanol is treated with a solution of 690 mg of potassium carbonate in 5 ml of water. The reaction mixture is refluxed under nitrogen atmosphere for 30 minutes, cooled, and evaporated to dryness. The residue is taken up in 10 ml of 10% aqueous hydrochloric acid and 50 ml of water and the resultant mixture extracted with ethyl acetate (2 x 50 ml). The combined extracts are dried over magnesium sulfate and evaporated to dryness under reduced pressure. crystallization of the residue from ethyl acetate-hexane affords 238 mg (89%) of 5-p-toluoyl-1,2-dihydro-3H-pyrrolo[1,2-a]pyrole-1-carboxylic acid, m.p. 182-183°C.
D. A solution of 250 mg of isopropyl 5-p-toluoyl-1,2-dihydro-3H-pyrrolo[1,2-a]pyrrole-1-carboxylate in 8 ml of methanol is treated under an atmosphere of nitrogen, with a solution of 200 mg of sodium hydroxide in 1 ml of water, maintaining the reaction mixture at room temperature for 1.5 hours. The methanol is then removed under reduced pressure and the basic solution which remains is diluted with 5 ml of water and extracted with ether to remove any unsaponifiable product. The aqueous solution is acidified with 10% hydrochloric acid and extracted three times with ethyl acetate. The combined extracts are dried and evaporated to dryness under reduced pressure, and the residue crystallized from ethyl acetate-hexane, to give 5-p-toluoyl-1,2-dihydro-3H-pyrrolo[1,2-a]pyrrole-1-carboxylic acid.
E. By following the above methods using 1.1 to 5 molar equivalents of N,N-dimethyl-benzamide in place of N,N-dimethyl-p-toluamide, and monitoring the course of the reaction by t.l.c., there is obtained:
   isopropyl-5-benzoyl-1,2-dihydro-3H-pyrrolo[1,2-a]pyrrole-1-carboyxlate, a light yellow oil, having the following physical constants: 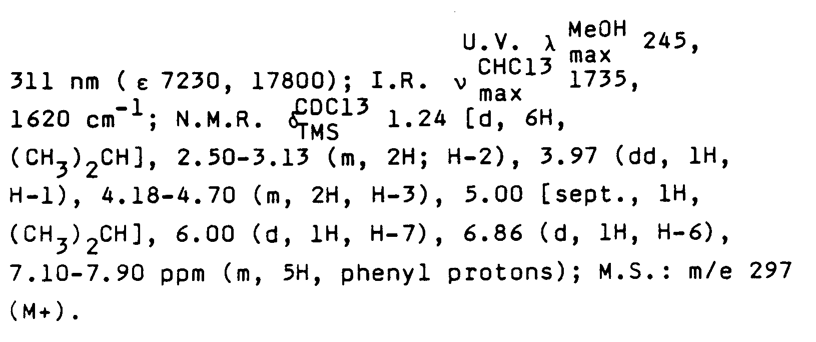 Upon hydrolysis of the isopropyl ester group, in accordance with the above methods, there is obtained: 5-benzoyl-1,2-dihydro-3H-pyrrolo[1,2-a]pyrrole-1-carboxylic acid, m.p. 160-161°C.
F. Benzmorpholide (1.45 g, 7.90 mmole), was placed in a 25 ml round bottom flask. POCl₃ (1.25 ml, 13.4 mmole) was added. This was stirred and heated in a 40° oil bath for 2.5 hours. A solution of methyl 1,2-dihydro-3H-pyrrolo[1,2-a]pyrrole-1-carboxylate (1.00 g, 6.10 mmole) in 3.4 ml CH₂Cl₂ was added and heating was continued for 2 hours, at this time, thin layer chromatography (TLC) of the reaction mixture showed reaction to be complete. The reaction mixture was added carefully to a solution of NaOH (3.09 g, 77.3 mmole) in 10 ml H₂O, allowing the CH₂Cl₂ to boil off, and the mixture heated to 50°. More NaOH (1.30 g, 32.5 mmole) was added. TLC 15 minutes later showed complete hydrolysis. The mixture was cooled and extracted with 2 x 10 ml CH₂Cl₂. The aqueous layer was acidified by the additionof 3.30 ml (39.6 mmole) conc. HCl. A milk formed. This was extracted with 5 x 10 ml CH₂Cl₂. Extract numbers 1-4 were dried (Na₂SO₄) and charcoaled (Darco® 660), then evaporated to a tan solid, which was a mixture of all benzoyl isomers. This solid was dissolved in 10 ml 2-propanol and hexane (10 ml) was added. A crystalline solid formed slowly. The first crop had a weight of 0.90 g (58.5%) and was pure (by TLC) 5-benzoyl-1,2-dihydro-3H-pyrrolo[1,2-a]pyrrole-1-carboxylic acid, m.p. 160-161°C.
G. A solution of 200 mg of 5-benzoyl-1,2-dihydro-3H-pyrrolo[1,2-a]pyrrole-1-carboxylic acid in 5 ml of dichloromethane is treated with an excess of ethereal diazomethane, and the reaction mixture is maintained at room temperature for 30 minutes. The solvents and excess reagent are eliminated under reduced pressure and the residue crystallized from ethyl acetate-methanol, to yield methyl 5-benzoyl-1,2-dihydro-3H-pyrrolo[1,2-a]pyrrole-1-carboxylate.
H. A solution of 300 mg of 5-p-toluoyl-1,2-dihydro-3H-pyrrolo[1,2-a]pyrrole-1-carboxylic acid in 5 ml of isoamyl alcohol is saturated with hydrogen chloride. After 24 hours, the excess alcohol is distilled off in vacuo and the residue purified by chromatography on alumina, to yield isoamyl 5-p-toluoyl-1,2-dihydro-3H-pyrrolo[1,2-a]pyrrole-1-carboxylate.
I. To a solution of 300 mg of 5-p-toluoyl-1,2-dihydro-3H-pyrrolo[1,2-a]pyrrole-1-carboxylic acid in 5 ml of methanol is added 1 molar equivalent of sodium hydroxide, in the form of a 0.1N solution. The solvent is then evaporated under reduced pressure and the residue taken up in 2 ml of methanol, followed by precipitation with ether, to yield crude sodium 5-p-toluoyl-1,2-dihydro-3H-pyrrolo[1,2-a]pyrrole-1-carboxylate which can be crystallized from ethyl acetate-hexane.
J. To a solution of 175 mg of 5-p-toluoyl-1,2-dihydro-3H-pyrrolo[1,2-a]pyrrole-1-carboxylic acid in 5 ml of methanol is added 1 molar equivalent of potassium hydroxide, in the form of a 0.1N solution, thus yielding a solution containing potassium 5-p-toluoyl-1,2-dihydro-3H-pyrrolo[1,2-a]pyrrole-1-carboxylate. A solution of 40 mg of calcium carbonate dissolved in the minimum amount of 1N hydrochloric acid necessary to effect solution of the calcium carbonate, is buffered with 100 mg of solid ammonium chloride, followed by the further addition of 5 ml of water. The thus obtained buffered calcium solution is then added to the solution of potassium 5-p-toluoyl-1,2-dihydro-3H-pyrrolo[1,2-a]pyrrole-1-carboxylate and the precipitate with forms is collected by filtration, washed with water and air dried, to yield calcium 5-p-toluoyl-1,2-dihydro-3H-pyrrolo[1,2-a]-pyrrole-1-carboxylate.
K. A solution of 200 mg of 5-p-toluoyl-1,2-dihydro-3H-pyrrolo[1,2-a]pyrrole-1-carboxylic acid in 15 ml of hot benzene is treated with 60 mg of isopropylamine. The solution is allowed to cool to room temperature and the product filtered off, washed with ether and dried to yield the isopropylamine salt of 5-p-toluoyl-1,2-dihydro-3H-pyrrolo[1,2-a]pyrrole-1-carboxylic acid.
L. 100 mg of sodium 5-benzoyl-1,2-dihydro-3H-pyrrolo[1,2-a]pyrrole-1-carboxylate is dissolved in 50 ml of water. 10 ml of concentrated hydrochloric acid are added with stirring at room temperature. The aqueous solution is then extracted with 2 x 50 ml portions of ethyl acetate, the organic extracts combined, and dried and evaporated. The product, 5-benzoyl-1,2-dihydro-3H-pyrrolo[1,2-a]pyrrole-1-carboxylic acid, is then recrystallized from ethanol/ether, m.p. 160-161°C.

## Claims (Claims for the following Contracting State(s): AT, BE, CH, DE, FR, GB, IT, LI, LU, NL, SE)

1. A compound of formula (XII), 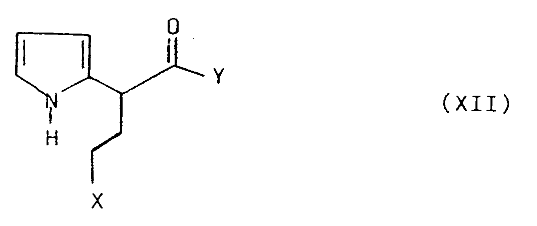 in which
X is halogen; and
Y is OH;
O⁻M⁺, wherein M is an alkali metal; or
NRR', wherein R is lower alkyl of 1 to 6 carbon atoms and R' is lower alkyl of 1 to 6 carbon atoms or aryl, or NRR' is the residue of a saturated cyclic amine containing 5 or 6 ring atoms, wherein aryl is substituted or unsubstituted phenyl, 2- or 3-furyl, 2- or 3-thienyl, or 2- or 3-pyrryl which, if substituted, can have one or more lower alkyl of 1 to 6 carbon atoms, lower alkoxy of 1 to 6 carbon atoms, or halo groups in any available position on the ring.

2. The compound of Claim 1 wherein X is chlorine.

3. The compound of Claim 1 wherein Y is NRR'.

4. The compound of Claim 2 wherein Y is NRR' and R and R' are each lower alkyl of 1 to 6 carbon atoms.

5. A process for producing a compound of formula (IX), 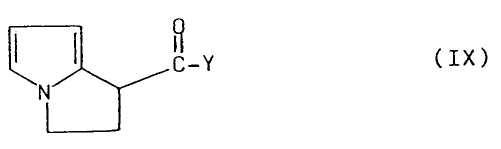 in which Y is as defined in Claim 1,
which comprises the reaction of a compound of formula (XII), 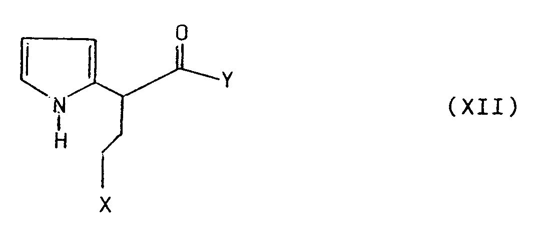 in which X and Y are as defined in Claim 1,
with a strong base in an aprotic polar solvent.

6. The process of Claim 5 wherein Y is NRR'.

7. The process of Claim 5 wherein Y is O⁻M⁺ or NRR', which further comprises the step of hydrolyzing the compound of formula (IX, Y = O⁻M⁺ or NRR') to afford a compound of formula (IX, Y = OH).

8. A process for producing a compound of formula (XII), 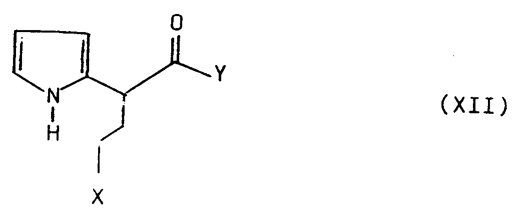 in which X and Y are as defined in Claim 1,
which comprises the reaction of a compound of formula X, 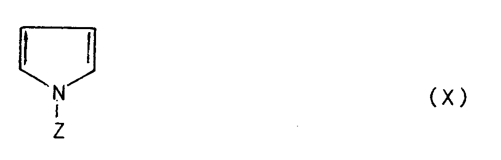 in which Z is Li, MgCl, or MgBr,
with a compound of formula XI, 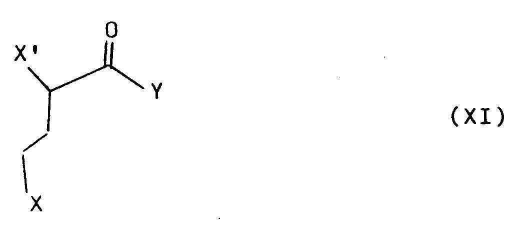 in which
X and Y are as defined in Claim 1 and X' is halogen.

9. A process for producing a compound of formula (I) or (IA) 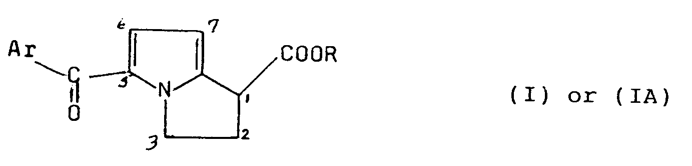 wherein Ar is substituted or unsubstituted phenyl, 2- or 3-furyl, 2- or 3-thienyl, or 2- or 3-pyrryl which, if substituted, can have one or more lower alkyl of 1 to 6 carbon atoms, lower alkoxy of 1 to 6 carbon atoms, or halo groups in any available position on the ring, and R is hydrogen (formula I) or alkyl of 1 to 12 carbon atoms (formula IA),
which comprises
(a) reacting a compound of formula (XII) 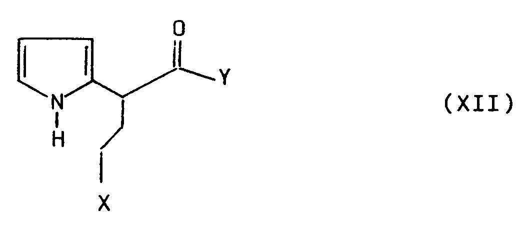 in which X and Y are as defined in Claim 1,
with a strong base in an aprotic polar solvent to form a compound of formula (IX) 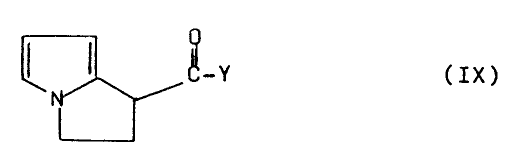 in which Y is as defined in Claim 1,
(b) hydrolyzing, if applicable, a compound of formula (IX) wherein Y is O⁻M⁺ or NRR' to afford a compound of formula (IX) wherein Y is OH;
(c) esterifying a compound of formula (IX) wherein Y is OH to form a compound of formula (IX) wherein Y is OR wherein R is alkyl of 1 to 12 carbon atoms; and
(d) aroylation of an ester of formula (IX) with an amide or morpholide, if appropriate, followed by hydrolysis, to form a compound of formula (I) or (IA).

10. The process of Claim 9 which further comprises one or more of the steps:
(a) converting a compound of formula (I) to a pharmaceutically acceptable salt of a compound of formula (I); or
(b) converting a salt of a compound of formula (I) to the corresponding free compound of formula (I); or
(c) esterifying a compound of formula (I); or
(d) converting an ester of formula (IA) to the corresponding free compound of formula (I).

11. The process of Claims 9 or 10 wherein the compound formed is 5-benzoyl-1,2-dihydro-3H-pyrrolo[1,2-a]pyrrole- 1-carboxylic acid or a pharmaceutically acceptable salt or ester thereof.

12. The process of Claims 9 or 10 wherein the compound formed is 5-p-anisoyl-1,2-dihydro-3H-pyrrolo[1,2-a]- pyrrole-1-carboxylic acid or a pharmaceutically acceptable salt or ester thereof.

## Claims (Claims for the following Contracting State(s): ES, GR)

1. A process for producing a compound of formula (XII) 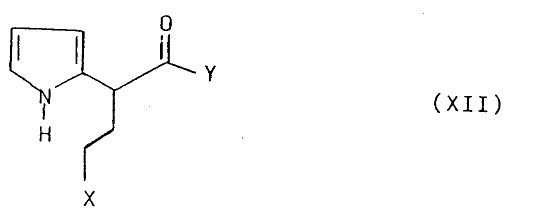 in which
X is halogen; and
Y is OH;
O⁻M⁺, wherein M is an alkali metal; or
NRR', wherein R is lower alkyl of 1 to 6 carbon atoms and R' is lower alkyl of 1 to 6 carbon atoms or aryl, or NRR' is the residue of a saturated cyclic amine containing 5 or 6 ring atoms, wherein aryl is substituted or unsubstituted phenyl, 2- or 3-furyl, 2- or 3-thienyl, or 2- or 3-pyrryl which, if substituted, can have one or more lower alkyl of 1 to 6 carbon atoms, lower alkoxy of 1 to 6 carbon atoms, or halo groups in any available position on the ring,
which comprises the reaction of a compound of formula X, 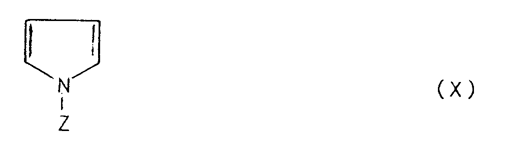 in which Z is Li, MgCl, or MgBr,
with a compound of formula XI, 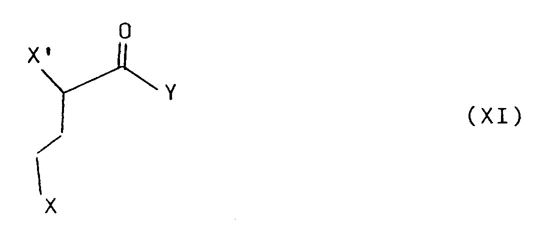 in which
X and Y are as defined above and X' is halogen.

2. The process of Claim 1 wherein X is chlorine.

3. The process of Claim 1 wherein Y is NRR'.

4. The process of Claim 2 wherein Y is NRR' and R and R' are each lower alkyl of 1 to 6 carbon atoms.

5. A process for producing a compound of formula (IX), 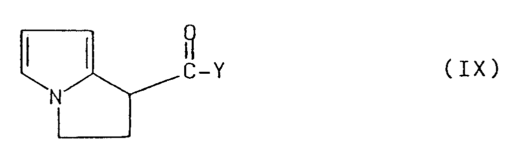 in which Y is as defined in Claim 1,
which comprises the reaction of a compound of formula (XII), 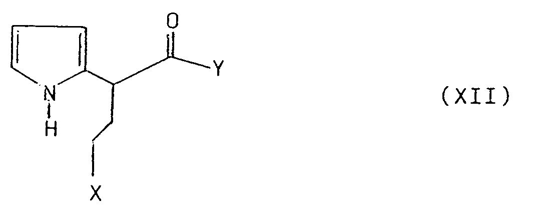 in which X and Y are as defined in Claim 1,
with a strong base in an aprotic polar solvent.

6. The process of Claim 5 wherein Y is NRR'.

7. The process of Claim 5 wherein Y is O⁻M⁺ or NRR', which further comprises the step of hydrolyzing the compound of formula (IX, Y = O⁻M⁺ or NRR') to afford a compound of formula (IX, Y = OH).

8. A process for producing a compound of formula (I) or (IA) 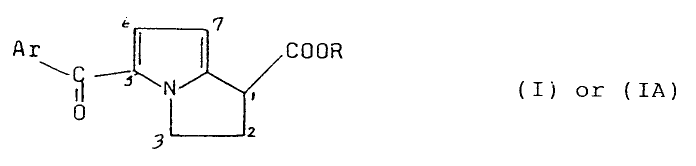 wherein Ar is substituted or unsubstituted phenyl, 2- or 3-furyl, 2- or 3-thienyl, or 2- or 3-pyrryl which, if substituted, can have one or more lower alkyl of 1 to 6 carbon atoms, lower alkoxy of 1 to 6 carbon atoms, or halo groups in any available position on the ring, and R is hydrogen (formula I) or alkyl of 1 to 12 carbon atoms (formula IA),
which comprises
(a) reacting a compound of formula (XII) 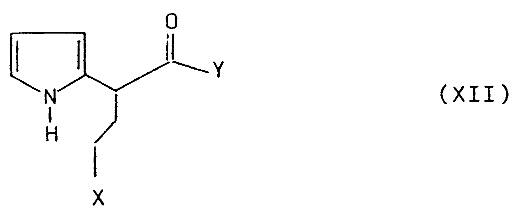 in which X and Y are as defined in Claim 1,
with a strong base in an aprotic polar solvent to form a compound of formula (IX) 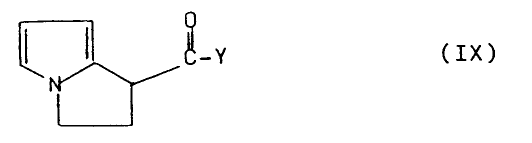 in which Y is as defined in Claim 1,
(b) hydrolyzing, if applicable, a compound of formula (IX) wherein Y is O⁻M⁺ or NRR' to afford a compound of formula (IX) wherein Y is OH;
(c) esterifying a compound of formula (IX) wherein Y is OH to form a compound of formula (IX) wherein Y is OR wherein R is alkyl of 1 to 12 carbon atoms; and
(d) aroylation of an ester of formula (IX) with an amide or morpholide, if appropriate, followed by hydrolysis, to form a compound of formula (I) or (IA).

9. The process of Claim 8 which further comprises one or more of the steps:
(a) converting a compound of formula (I) to a pharmaceutically acceptable salt of a compound of formula (I); or
(b) converting a salt of a compound of formula (I) to the corresponding free compound of formula (I); or
(c) esterifying a compound of formula (I); or
(d) converting an ester of formula (IA) to the corresponding free compound of formula (I).

10. The process of Claims 8 or 9 wherein the compound formed is 5-benzoyl-1,2-dihydro-3H-pyrrolo[1,2-a]pyrrole- 1-carboxylic acid or a pharmaceutically acceptable salt or ester thereof.

11. The process of Claims 8 or 9 wherein the compound formed is 5-p-anisoyl-1-2-dihydro-3H-pyrrolo[1,2-a]- pyrrole-1-carboxylic acid or a pharmaceutically acceptable salt or ester thereof.

## Patentansprüche (Patentansprüche für folgende(n) Vertragsstaat(en): AT, BE, CH, DE, FR, GB, IT, LI, LU, NL, SE)

1. Verbindung der Formel (XII), 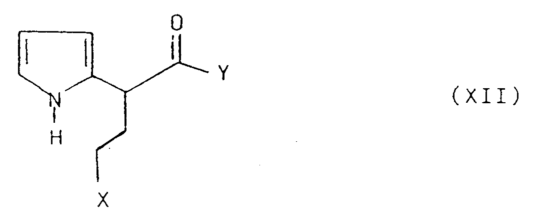 in der
X Halogen ist; und
Y OH;
O⁻M⁺, worin M ein Alkalimetall ist; oder NRR' ist, worin R Niederalkyl mit 1 bis 6 Kohlenstoffatomen ist und R' Niederalkyl mit 1 bis 6 Kohlenstoffatomen oder Aryl ist oder NRR' der Rest eines gesättigten cyclischen Amins, der 5 oder 6 Ringatome enthält, ist, worin Aryl substituiertes oder unsubstituiertes Phenyl, 2- oder 3-Furyl, 2- oder 3-Thienyl oder 2- oder 3-Pyrryl ist, welche, falls sie substituiert sind, an irgendeiner verfügbaren Position am Ring eine oder mehrere Niederalkylgruppen mit 1 bis 6 Kohlenstoffatomen, Niederalkoxygruppen mit 1 bis 6 Kohlenstoffatomen oder Halogengruppen aufweisen können.

2. Verbindung nach Anspruch 1, worin X Chlor ist.

3. Verbindung nach Anspruch 1, worin Y NRR' ist.

4. Verbindung nach Anspruch 2, worin Y NRR' ist und R und R' jeweils Niederalkyl mit 1 bis 6 Kohlenstoffatomen sind.

5. Verfahren zur Herstellung einer Verbindung der Formel (IX), 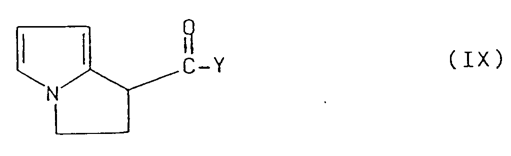 in der Y wie in Anspruch 1 definiert ist, umfassend das Umsetzen einer Verbindung der Formel (XII), 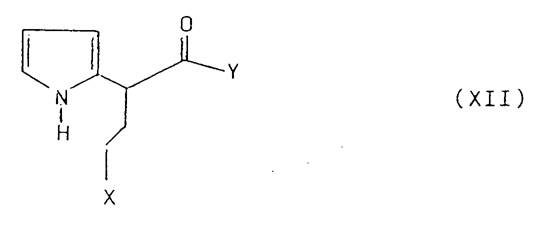 in der X und Y wie in Anspruch 1 definiert sind, mit einer starken Base in einem aprotischen polaren Lösungsmittel.

6. Verfahren nach Anspruch 5, worin Y NRR' ist.

7. Verfahren nach Anspruch 5, worin Y O⁻M⁺ oder NRR' ist, welches weiter umfaßt den Schritt des Hydrolysierens der Verbindung der Formel (IX, Y = O⁻M⁺ oder NRR'), um eine Verbindung der Formel (IX, Y = OH) zu liefern.

8. Verfahren zur Herstellung einer Verbindung der Formel (XII), 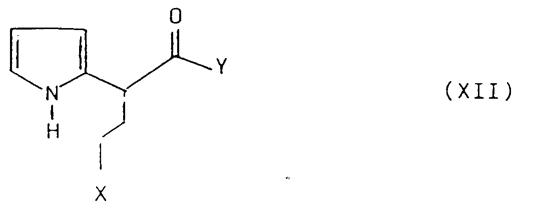 in der X und Y wie in Anspruch 1 definiert sind,
umfassend das Umsetzen einer Verbindung der Formel X, 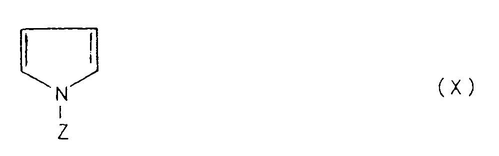 in der Z Li, MgCl oder MgBr ist,
mit einer Verbindung der Formel XI, 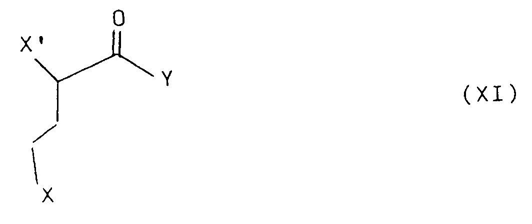 in der
X und Y wie in Anspruch 1 definiert sind und X' Halogen ist.

9. Verfahren zur Herstellung einer Verbindung der Formel (I) oder (IA), 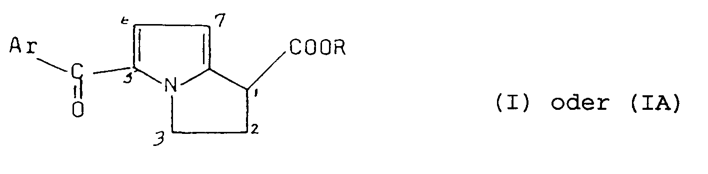 worin Ar substituiertes oder unsubstituiertes Phenyl, 2- oder 3-Furyl, 2- oder 3-Thienyl oder 2- oder 3-Pyrryl ist, welche, falls sie substituiert sind, an irgendeiner verfügbaren Position des Rings eine oder mehrere Niederalkylgruppen mit 1 bis 6 Kohlenstoffatomen, Niederalkoxygruppen mit 1 bis 6 Kohlenstoffatomen oder Halogengruppen aufweisen können, und R Wasserstoff (Formel I) oder Alkyl mit 1 bis 12 Kohlenstoffatomen (Formel IA), ist,
umfassend
(a) das Umsetzen einer Verbindung der Formel (XII), 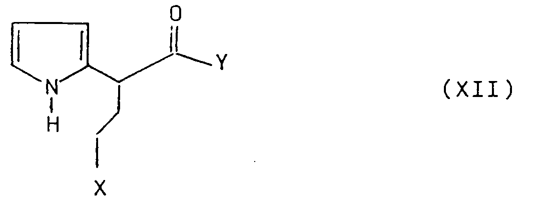 in der X und Y wie in Anspruch 1 definiert sind, mit einer starken Base in einem aprotischen polaren Lösungsmittel, um eine Verbindung der Formel (IX) zu bilden, 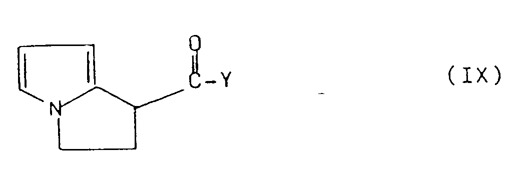 in der Y wie in Anspruch 1 definiert ist,
(b) das Hydrolysieren, falls anwendbar, einer Verbindung der Formel (IX), in der Y O⁻M⁺ oder NRR' ist, um eine Verbindung der Formel (IX) zu liefern, in der Y OH ist;
(c) das Verestern einer Verbindung der Formel (IX), in der Y OH ist, um eine Verbindung der Formel (IX) zu bilden, in der Y OR ist, worin R Alkyl mit 1 bis 12 Kohlenstoffatomen ist; und
(d) das Aroylieren eines Esters der Formel (IX) mit einem Amid oder Morpholid, falls angezeigt, gefolgt von Hydrolyse, um eine Verbindung der Formel (I) oder (IA) zu bilden.

10. Verfahren nach Anspruch 9, welches weiter umfaßt einen oder mehrere der Schritte:
(a) Überführen einer Verbindung der Formel (I) in ein pharmazeutisch annehmbares Salz einer Verbindung der Formel (I); oder
(b) Überführen eines Salzes einer Verbindung der Formel (I) in die entsprechende freie Verbindung der Formel (I); oder
(c) Verestern einer Verbindung der Formel (I); oder
(d) Überführen eines Esters der Formel (IA) in die entsprechende freie Verbindung der Formel (I).

11. Verfahren nach Anspruch 9 oder 10, worin die gebildete Verbindung 5-Benzoyl-1,2-dihydro-3H-pyrrolo[1,2-a]pyrrol-1-carbonsäure oder ein pharmazeutisch annehmbares Salz oder ein pharmazeutisch annehmbarer Ester derselben ist.

12. Verfahren nach den Ansprüchen 9 oder 10, worin die gebildete Verbindung 5-p-Anisoyl-1,2-dihydro-3H-pyrrolo[1,2-a]-pyrrol-1-carbonsäure oder ein pharmazeutisch annehmbares Salz oder ein pharmazeutisch annehmbarer Ester derselben ist.

## Patentansprüche (Patentansprüche für folgende(n) Vertragsstaat(en): ES, GR)

1. Verfahren zur Herstellung einer Verbindung der Formel (XII), 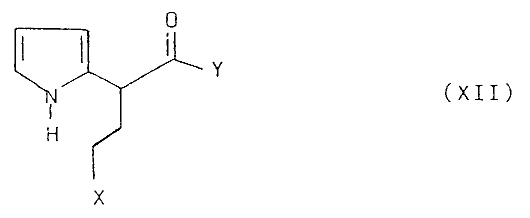 in der
X Halogen ist; und
Y OH;
O⁻M⁺, worin M ein Alkalimetall ist; oder NRR' ist, worin R Niederalkyl mit 1 bis 6 Kohlenstoffatomen ist und R' Niederalkyl mit 1 bis 6 Kohlenstoffatomen oder Aryl ist oder NRR' der Rest eines gesättigten cyclischen Amins, der 5 oder 6 Ringatome enthält, ist, worin Aryl substituiertes oder unsubstituiertes Phenyl, 2- oder 3-Furyl, 2- oder 3-Thienyl oder 2- oder 3-Pyrryl ist, welche, falls sie substituiert sind, an irgendeiner verfügbaren Position am Ring eine oder mehrere Niederalkylgruppen mit 1 bis 6 Kohlenstoffatomen, Niederalkoxygruppen mit 1 bis 6 Kohlenstoffatomen oder Halogengruppen aufweisen können,
umfassend das Umsetzen einer Verbindung der Formel X, 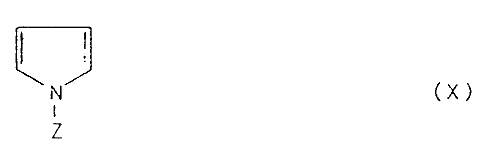 in der Z Li, MgCl oder MgBr ist,
mit einer Verbindung der Formel XI, 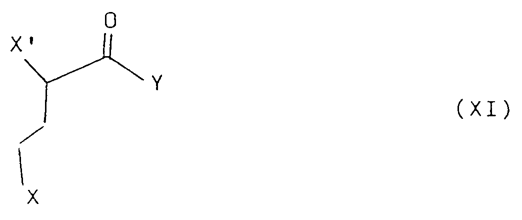 in der
X und Y wie oben definiert sind und X' Halogen ist.

2. Verfahren nach Anspruch 1, worin X Chlor ist.

3. Verfahren nach Anspruch 1, worin Y NRR' ist.

4. Verfahren nach Anspruch 2, worin Y NRR' ist und R und R' jeweils Niederalkyl mit 1 bis 6 Kohlenstoffatomen sind.

5. Verfahren zur Herstellung einer Verbindung (IX), 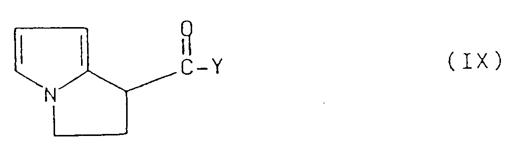 in der Y wie in Anspruch 1 definiert ist,
umfassend das Umsetzen einer Verbindung der Formel (XII), 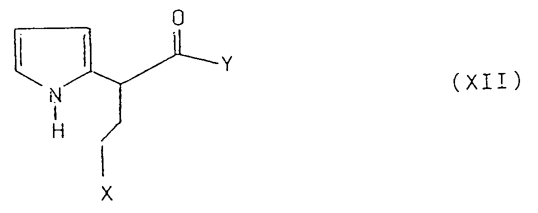 in der X und Y wie in Anspruch 1 definiert sind, mit einer starken Base in einem aprotischen polaren Lösungsmittel.

6. Verfahren nach Anspruch 5, worin Y NRR' ist.

7. Verfahren nach Anspruch 5, worin Y O⁻M⁺ oder NRR' ist, welches weiter umfaßt den Schritt des Hydrolysierens der Verbindung der Formel (IX, Y = O⁻M⁺ oder NRR'), um eine Verbindung der Formel (IX, Y = OH) zu liefern.

8. Verfahren zur Herstellung einer Verbindung der Formel (I) oder (IA), 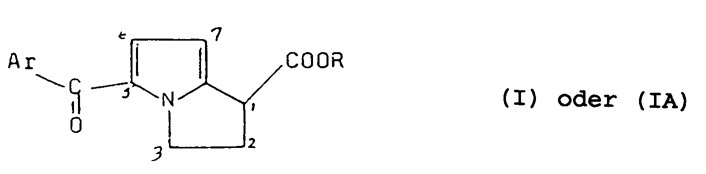 worin Ar substituiertes oder unsubstituiertes Phenyl, 2- oder 3-Furyl, 2- oder 3-Thienyl oder 2- oder 3-Pyrryl ist, welche, falls sie substituiert sind, an irgendeiner verfügbaren Position am Ring eine oder mehrere Niederalkylgruppen mit 1 bis 6 Kohlenstoffatomen, Niederalkoxygruppen mit 1 bis 6 Kohlenstoffatomen oder Halogengruppen aufweisen können, und R Wasserstoff (Formel I) oder Aklyl mit 1 bis 12 Kohlenstoffatomen (Formel IA) ist,
umfassend
(a) das Umsetzen einer Verbindung der Formel (XII), 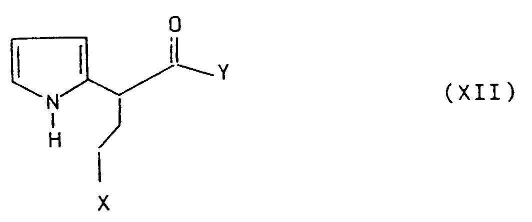 in der X und Y wie in Anspruch 1 definiert sind, mit einer starken Base in einem aprotischen polaren Lösungsmittel, um eine Verbindung der Formel (IX) zu bilden, 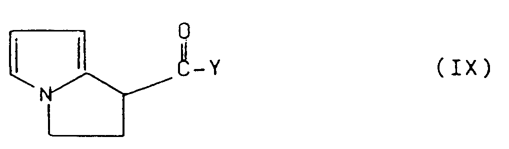 in der Y wie in Anspruch 1 definiert ist,
(b) das Hydrolysieren, falls anwendbar, einer Verbindung der Formel (IX), in der Y O⁻M⁺ oder NRR' ist, um eine Verbindung der Formel (IX) zu liefern, in der Y OH ist;
(c) das Verestern einer Verbindung der Formel (IX), in der Y OH ist, um eine Verbindung der Formel (IX) zu bilden, in der Y OR ist, worin R Alkyl mit 1 bis 12 Kohlenstoffatomen ist; und
(d) das Aroylieren eines Esters der Formel (IX) mit einem Amid oder Morpholid, falls angezeigt, gefolgt von Hydrolyse, um eine Verbindung der Formel (I) oder (IA) zu bilden.

9. Verfahren nach Anspruch 8, welches weiter umfaßt einen oder mehrere der Schritte:
(a) Überführen einer Verbindung der Formel (I) in ein pharmazeutisch annehmbares Salz einer Verbindung der Formel (I); oder
(b) Überführen eines Salzes einer Verbindung der Formel (I) in die entsprechende freie Verbindung der Formel (I); oder
(c) Verestern einer Verbindung der Formel (I); oder
(d) Überführen eines Esters der Formel (IA) in die entsprechende freie Verbindung der Formel (I).

10. Verfahren nach Anspruch 8 oder 9, worin die gebildete Verbindung 5-Benzoyl-1,2-dihydro-3H-pyrrolo[1,2-a]pyrrol-1-carbonsäure oder ein pharmazeutisch annehmbares Salz oder ein pharmazeutisch annehmbarer Ester derselben ist.

11. Verfahren nach den Ansprüchen 8 oder 9, worin die gebildete Verbindung 5-p-Anisoyl-1,2-dihydro-3H-pyrrolo[1,2-a]-pyrrol-1-carbonsäure oder ein pharmazeutisch annehmbares Salz oder ein pharmazeutisch annehmbarer Ester derselben ist.

## Revendications (Revendications pour l'(les) Etat(s) contractant(s) suivant(s): AT, BE, CH, DE, FR, GB, IT, LI, LU, NL, SE)

1. Composé de formule (XII) 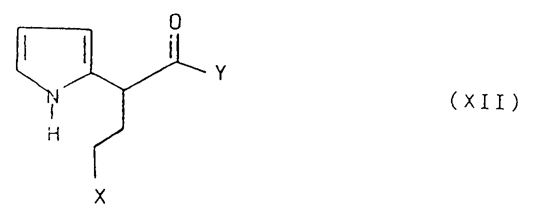 dans laquelle
X représente un halogène ; et
Y représente un groupe OH ;
O⁻M⁺, dans lequel M représente un métal alcalin ; ou NRR', dans lequel R représente un groupe alkyle inférieur ayant 1 à 6 atomes de carbone et R' représente un groupe alkyle inférieur ayant 1 à 6 atomes de carbone ou aryle, ou bien NRR' représente le résidu d'une amine cyclique saturée contenant 5 ou 6 atomes dans le cycle, le groupe aryle étant un groupe phényle, 2- ou 3-furyle, 2- ou 3-thiényle, ou 2- ou 3-pyrryle substitué ou non substitué qui, s'il est substitué, peut porter un ou plusieurs groupes alkyle inférieurs ayant 1 à 6 atomes de carbone, alkoxy inférieurs ayant 1 à 6 atomes de carbone ou halogéno dans n'importe quelle position disponible sur le noyau.

2. Composé suivant la revendication 1, dans lequel X représente le chlore.

3. Composé suivant la revendication 1, dans lequel Y représente un groupe NRR'.

4. Composé suivant la revendication 2, dans lequel Y représente un groupe NRR' et R et R' représentent chacun un groupe alkyle inférieur ayant 1 à 6 atomes de carbone.

5. Procédé de production d'un composé de formule (IX), 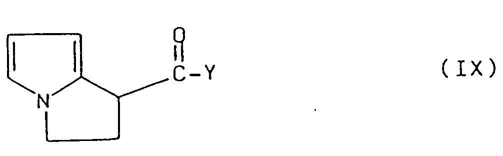 dans laquelle Y répond à la définition figurant dans la revendication 1,
qui comprend la réaction d'un composé de formule (XII) 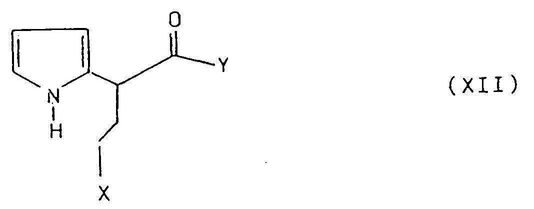 dans laquelle X et Y répondent aux définitions suivant la revendication 1,
avec une base forte dans un solvant polaire aprotique.

6. Procédé suivant la revendication 5, dans lequel Y représente un groupe NRR'.

7. Procédé suivant la revendication 5, dans lequel Y représente un groupe O⁻M⁺ ou NRR', qui comprend en outre l'étape d'hydrolyse du composé de formule (IX, Y = O⁻M⁺ ou NRR'), ce qui donne un composé de formule (IX, Y = OH).

8. Procédé de production d'un composé de formule (XII) 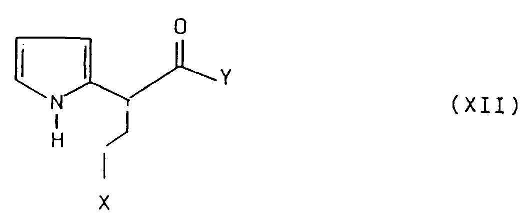 dans laquelle X et Y répondent aux définitions suivant la revendication 1,
qui comprend la réaction d'un composé de formule X 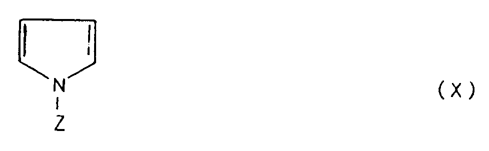 dans laquelle Z représente Li, MgCl ou MgBr,
avec un composé de formule XI, 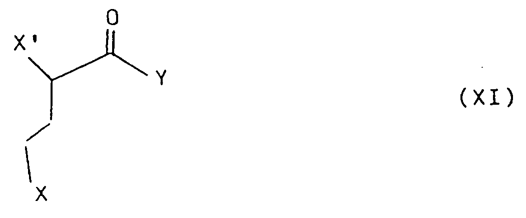 dans laquelle
X et Y répondent aux définitions suivant la revendication 1 et X' représente un halogène.

9. Procédé de production d'un composé de formule (I) ou (IA) 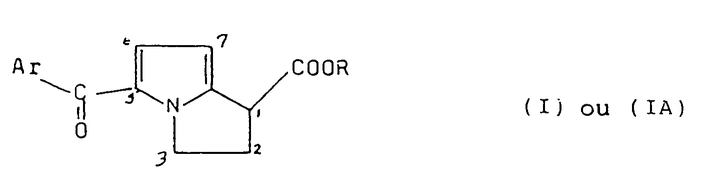 dans laquelle Ar représente un groupe phényle , 2- ou 3-furyle, 2- ou 3-thiényle ou 2- ou 3-pyrryle substitué ou non substitué qui, s'il est substitué, peut porter un ou plusieurs groupes alkyle ayant 1 à 6 atomes de carbone, alkoxy inférieurs ayant 1 à 6 atomes de carbone ou halogéno dans n'importe quelle position disponible sur le noyau, et R représente l'hydrogène (formule I) ou un groupe alkyle ayant 1 à 12 atomes de carbone (formule IA),
qui comprend
(a) la réaction d'un composé de formule (XII) 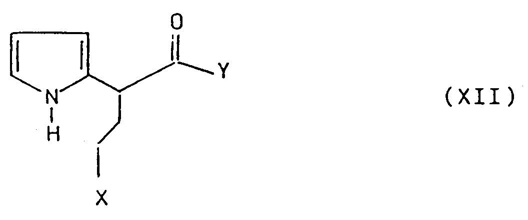 dans laquelle X et Y répondent aux définitions suivant la revendication 1,
avec une base forte dans un solvant polaire aprotique pour former un composé de formule (IX) 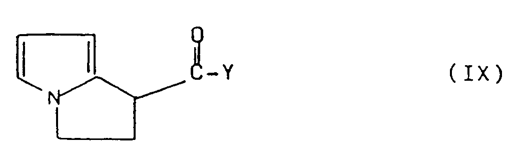 dans laquelle Y répond à la définition suivant la revendication 1,
(b) l'hydrolyse, si cela est possible, d'un composé de formule (IX) dans laquelle Y réprésente un groupe O⁻M⁺ ou NRR', ce qui donne un composé de formule (IX) dans laquelle Y représente un groupe OH ;
(c) l'estérification d'un composé de formule (IX) dans laquelle Y représente un groupe OH pour former un composé de formule (IX) dans laquelle Y représente un groupe OR dans lequel R est un groupe alkyle ayant 1 à 12 atomes de carbone ; et
(d) l'aroylation d'un ester de formule (IX) avec un amide ou morpholide, si cela est approprié, suivie par une hydrolyse, donnant un composé de formule (I) ou (IA).

10. Procédé suivant la revendication 9, qui comprend en outre une ou plusieurs des étapes suivantes :
(a) transformation d'un composé de formule (I) en un sel pharmaceutiquement acceptable d'un composé de formule (I) ;
(b) transformation d'un sel d'un composé de formule (I) en le composé libre correspondant de formule (I) ;
(c) estérification d'un composé de formule (I) ;
(d) transformation d'un ester de formule (IA) en le composé libre correspondant de formule (I).

11. Procédé suivant la revendication 9 ou 10, dans lequel le composé formé est l'acide 5-benzoyl-1,2-dihydro-3H-pyrrolo[1,2-a]pyrrole-1-carboxylique ou un de ses sels ou esters pharmaceutiquement acceptables.

12. Procédé suivant la revendication 9 ou 10, dans lequel le composé formé est l'acide 5-p-anisoyl-1,2-dihydro-3H-pyrrolo[1,2-a]-pyrrole-1-carboxylique ou un de ses sels ou esters pharmaceutiquement acceptables.

## Revendications (Revendications pour l'(les) Etat(s) contractant(s) suivant(s): ES, GR)

1. Procédé de production d'un composé de formule (XII) 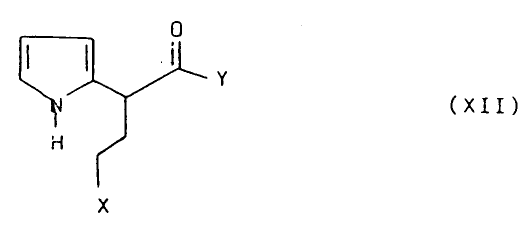 dans laquelle
X représente un halogène ; et
Y représente un groupe OH ;
O⁻M⁺, dans lequel M représente un métal alcalin ; ou NRR', dans lequel R représente un groupe alkyle inférieur ayant 1 à 6 atomes de carbone et R' représente un groupe alkyle inférieur ayant 1 à 6 atomes de carbone ou aryle, ou bien NRR' est le résidu d'une amine cyclique saturée contenant 5 ou 6 atomes dans le cycle, le groupe aryle étant un groupe phényle, 2- ou 3-furyle, 2- ou 3-thiényle , ou 2- ou 3-pyrryle substitué ou non substitué qui, s'il est substitué, peut porter un ou plusieurs groupes alkyle inférieurs ayant 1 à 6 atomes de carbone, alkoxy inférieurs ayant 1 à 6 atomes de carbone ou halogéno dans n'importe quelle position disponible sur le noyau,
qui comprend la réaction d'un composé de formule X, 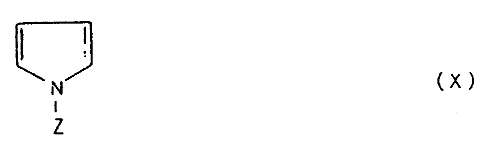 dans laquelle Z représente Li, MgCl ou MgBr, avec un composé de formule XI, 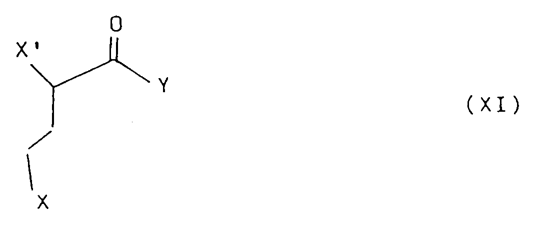 dans laquelle
X et Y répondent aux définitions précitées et X' représente un halogène.

2. Procédé suivant la revendication 1, dans lequel X représente le chlore.

3. Procédé suivant la revendication 1, dans lequel Y représente un groupe NRR'.

4. Procédé suivant la revendication 2, dans lequel Y représente un groupe NRR', et R et R' représentent chacun un groupe alkyle inférieur ayant 1 à 6 atomes de carbone.

5. Procédé de production d'un composé de formule (IX), 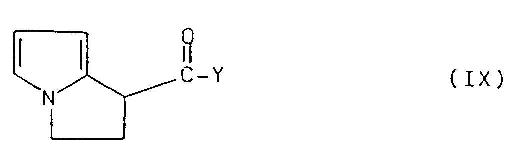 dans laquelle Y répond à la définition suivant la revendication 1,
qui comprend la réaction d'un composé de formule (XII) 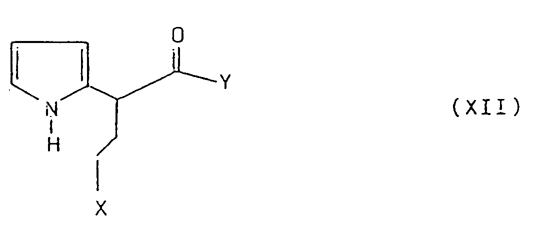 dans laquelle X et Y répondent aux définitions suivant la revendication 1,
avec une base forte dans un solvant polaire aprotique.

6. Procédé suivant la revendication 5, dans lequel Y représente un groupe NRR'.

7. Procédé suivant la revendication 5, dans lequel Y représente un groupe O⁻M⁺ ou NRR', qui comprend en outre l'étape d'hydrolyse du composé de formule (IX, Y = O⁻M⁺ ou NRR'), ce qui donne un composé de formule (IX, Y = OH).

8. Procédé de production d'un composé de formule (I) ou (IA) 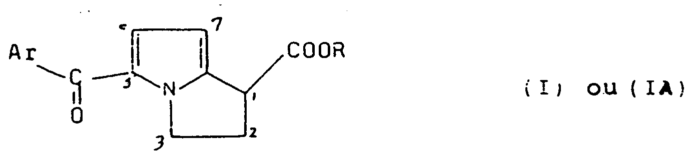 dans laquelle Ar représente un groupe phényle, 2- ou 3-furyle, 2- ou 3-thiényle ou 2- ou 3-pyrryle substitué ou non substitué qui, s'il est substitué, peut porter un ou plusieurs groupes alkyle inférieurs ayant 1 à 6 atomes de carbone, alkoxy inférieurs ayant 1 à 6 atomes de carbone ou halogéno dans n'importe quelle position disponible sur le noyau, et R représente l'hydrogène (formule I) ou un groupe alkyle ayant 1 à 12 atomes de carbone (formule IA), qui comprend
(a) la réaction d'un composé de formule (XII) 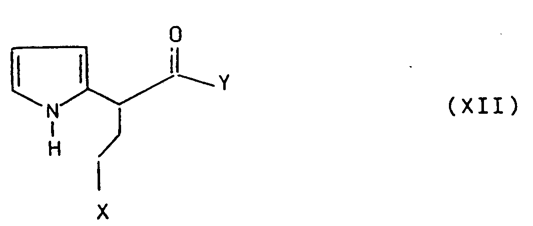 dans laquelle X et Y répondent aux définitions suivant la revendication 1,
avec une base forte dans un solvant polaire aprotique pour former un composé de formule (IX) 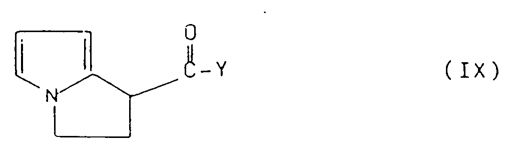 dans laquelle Y répond à la définition suivant la revendication 1,
(b) l'hydrolyse, si cela est réalisable, d'un composé de formule (IX) dans laquelle Y réprésente un groupe O⁻M⁺ ou NRR', ce qui donne un composé de formule (IX) dans laquelle Y représente un groupe OH ;
(c) l'estérification d'un composé de formule (IX) dans laquelle Y représente un groupe OH pour former un composé de formule (IX) dans laquelle Y représente un groupe OR dans lequel R est un groupe alkyle ayant 1 à 12 atomes de carbone ; et
(d) l'aroylation d'un ester de formule (IX) avec un amide ou morpholide, si cela est approprié, suivie par une hydrolyse, pour former un composé de formule (I) ou (IA).

9. Procédé suivant la revendication 8, qui comprend en outre une ou plusieurs des étapes suivantes :
(a) transformation d'un composé de formule (I) en un sel pharmaceutiquement acceptable d'un composé de formule (I) ;
(b) transformation d'un sel d'un composé de formule (I) en le composé libre correspondant de formule (I) ;
(c) estérification d'un composé de formule (I) ;
(d) transformation d'un ester de formule (IA) en le composé libre correspondant de formule (I).

10. Procédé suivant la revendication 8 ou 9, dans lequel le composé formé est l'acide 5-benzoyl-1,2-dihydro-3H-pyrrolo[1,2-a]pyrrole-1-carboxylique ou un de ses sels ou esters pharmaceutiquement acceptables.

11. Procédé suivant la revendication 8 ou 9, dans lequel le composé formé est l'acide 5-p-anisoyl-1,2-dihydro-3H-pyrrolo[1,2-a]-pyrrole-1-carboxylique ou un de ses sels ou esters pharmaceutiquement acceptables.
